# EUROPÄISCHE PATENTSCHRIFT

(11) **EP 3 730 097 B1**
(45) Veröffentlichungstag und Bekanntmachung des Hinweises auf die Patenterteilung: **03.11.2021**
(21) Anmeldenummer: 19171459.1
(22) Anmeldetag: 26.04.2019
(51) Int. Cl.: A61F 2/36

(54) **PROBE HALSSTÜCK FÜR EINE GELENKENDOPROTHESE**
TRIAL NECKPIECE FOR A JOINT ENDOPROSTHESIS
COMPOSANT DE PROTÉSESE DE COU D'ESSAI POUR ENDOPROTHÈSE D'ARTICULATION

(43) Veröffentlichungstag der Anmeldung: 28.10.2020
(73) Patentinhaber: Waldemar Link GmbH & Co. KG, 22339 Hamburg (DE)
(72) Erfinder: DMUSCHEWSKY, Klaus, 22397Hamburg (DE); ALTAN, Emel, 47807 Krefeld (DE)
(74) Vertreter: Glawe, Delfs, Moll

(56) Entgegenhaltungen:
- EP-A1- 0 145 641
- EP-A1- 0 283 706
- WO-A2-2007/028832
- FR-A1- 2 735 972
- US-A1- 2007 112 430

## Beschreibung

Die Erfindung betrifft ein Probe-Instrumentarium für eine Gelenkendoprothese, umfassend insbesondere ein Probe-Halsstück, das zur temporären Anordnung an einem in einen Röhrenknochen steckbaren gesonderten Schaftkörper ausgebildet ist.

Endoprothesen, und zwar insbesondere Gelenkendoprothesen, erfordern eine sichere Verankerung im Knochen, um so langzeitstabil und zuverlässig ihrer Funktion nachkommen zu können. Zur besseren Verankerung weisen derartige Gelenkendoprothesen daher einen langen Schaft auf. Dieser muss in dem Knochen, an dem die Gelenkendoprothese anzuordnen ist, sicher befestigt werden. Meist handelt sich hierbei um Röhrenknochen, wobei der Schaft in einen Markraum oder einen anderen zu schaffenden Hohlraum des Röhrenknochens einzubringen ist. Der benötigte Raum in dem Knochen wird in der Regel durch Ausräumen geschaffen, bspw. im Markkanal eines Femurknochens, insbesondere mittels einer Reibahle. Nach dem Ausräumen wird geprüft, ob Weite und Tiefe des geschaffenen Hohlraums ausreichend sind. Diese Prüfung ist wesentlich, da sie entscheidend ist hinsichtlich eines korrekten Sitzes der nachfolgend zu implantierenden Gelenkendoprothese.

Es ist bekannt, dafür gesonderte Probe-Instrumente bzw. -Implantate zu verwenden. Allerdings bedeutet die Verwendung derartiger gesonderter Probe-Instrumente bzw. -Implantate zusätzlichen Aufwand, und zwar sowohl bei der Bereitstellung wie auch anschließend bei der Reinigung nach der Operation. In der Praxis wird daher häufig dazu übergegangen, keine gesonderten Probe-Instrumente zu verwenden, sondern stattdessen das Instrument zu verwenden, welches zum Ausräumen des Hohlraums verwendet wurde (meist ist dies die Reibahle). Der Vorteil hierbei ist, dass dieses Instrument sich ohnehin bereits im Hohlraum befindet, und daher nicht noch gesondert eingebracht zu werden braucht. Ferner ist eventuell erforderliche Nacharbeit somit einfach durchzuführen, indem einfach mit der Reibahle dann weiterer Hohlraum geschaffen würde. Es ergeben sich so signifikante Handlingvorteile. Dem steht als Nachteil gegenüber, dass Instrumente wie die Reibahle an sich nicht dafür geschaffen sind, als Probe-Instrument zu fungieren. Bei Instrumenten wie der Reibahle steht vielmehr der eigentliche Arbeitszweck im Vordergrund. Das äußert sich u. a. darin, dass die Reibahle an ihrem oberen Ende einen Triebkopf aufweist, der ziemlich weit nach außen aus dem Knochen übersteht (um von dort die Reibahle betätigen zu können). Dieser weite Überstand ist beim Probieren störend und erschwert eine genaue Prüfung bzw. Ausrichtung.

Um dies zu vermeiden sind Systeme bekannt geworden, die über eine verhältnismäßig kurze Reibahle verfügen (oder über eine zweiteilige, deren oberer Teil abgenommen wird), und auf deren oberes Ende ein gesondertes Probe-Halsstück montierbar ist. Da je nach individueller Anatomie des Patienten die Höhe unterschiedlich ist, sind bei den bekannten Systemen, wie z.B. in Patent EP 0283706 A1 offenbart, Zwischenstücke vorgesehen oder es umfasst mehrere, verschieden hohe Probe-Halsstücke, von denen dann ein geeignetes auszuwählen ist. Zudem verlangen die bekannten Systeme häufig ein Verschrauben der einzelnen Elemente. Die Handhabung von Kleinteilen, wie Zwischenstücken oder Schrauben, ist umständlich und in Bezug auf das Verlustrisiko gefährlich, und das Verwenden von mehreren, verschieden hohen Probe-Halsstücken ist umständlich.

Der Erfindung liegt die Aufgabe zugrunde, ein verbessertes Instrumentarium zu schaffen, das diese Nachteile vermeidet.

Die erfindungsgemäße Lösung liegt in den Merkmalen der unabhängigen Ansprüche. Vorteilhafte Weiterbildungen sind Gegenstand der abhängigen Ansprüche.

Bei einem Probe-Halsstück für eine Gelenkendoprothese, das zur temporären Anordnung an einem in einen Röhrenknochen steckbaren gesonderten Schaftkörper (insbesondere einer Reibahle) ausgebildet ist, wobei das Probe-Halsstück umfasst: einen Befestigungsbereich, der mittels einer Steckverbindung auf ein Kopfteil des Schaftkörpers in mindestens einer definierten Position temporär aufsteckbar (und abnehmbar) ist, einen Halsbereich, der zur Aufnahme eines Gelenkelements einer Gelenkendoprothese ausgebildet ist, ist erfindungsgemäß vorgesehen, dass das Probe-Halsstück als ein gesondertes, steckbares Aufsatzstück ausgeführt ist, das mit seinem Befestigungsbereich formschlüssig auf das Kopfteil aufsteckbar und arretierbar ist, wobei eine Verrastungseinrichtung vorgesehen ist, die mehrere, vorzugsweise mindestens fünf, Raststufen umfasst für verschiedene Höhenstufen des Probe-Halsstücks am Kopfteil des Schaftkörpers.

Nachfolgend seien einige verwendete Begriffe erläutert:
Unter einem freien Ende eines Röhrenknochens wird das Ende verstanden, an dem die Endoprothese zu implantieren ist. So ist zum Beispiel bei der Implantation einer Hüftgelenk-Endoprothese das proximale Ende des Femurs das freie Ende des Röhrenknochens.

Unter einem Antriebskopf wird ein Koppelstück verstanden, dass an einem Ende eines Werkzeugs (bspw. einer Reibahle) angeordnet ist, und an dem dieses Werkzeug antreibende Kräfte angreifen. Es kann ein Handgriff angekoppelt werden, um das Werkzeug manuell zu betätigen, und/oder gegebenenfalls ein maschineller Antrieb.

Unter Höhenstufen wird verstanden eine unterschiedliche Positionierung entlang einer Längsachse eines Schaftkörpers. Es ergeben sich somit bei einer vertikalen Orientierung des Schaftkörpers unterschiedliche Höhen für eine Kombination aus Schaftkörper und Probe-Halsstück.

Die Erfindung beruht auf dem Gedanken, ein leicht aufsetzbares Probe-Halsstück zu schaffen, das in verschiedenen Höhenpositionen definiert angeordnet werden kann, um so zum einen eine sichere und definierte Anordnung zu erreichen und zum anderen das Einstellen unterschiedlicher Höhen zu ermöglichen. Damit wird es überflüssig, eine Mehrzahl von unterschiedlich hohen Probe-Halsstücken vorzusehen oder gesonderte Teile zur Höhenanpassung zu verwenden, wie Zwischenringe. Ein zusätzliches Auf- bzw. Umstecken anderer oder weiterer Teile zur Höhenanpassung kann somit erfindungsgemäß vermieden werden. Dank der Verrastung ist eine sichere und definierte Höheneinstellung erreicht, und zwar in verschiedenen durch die Raststufen definierten Höhen. Damit wird die Handhabung nicht nur erheblich vereinfacht, sondern auch sicherer (da keine weiteren Teile, insbesondere Kleinteile, benötigt werden, mit der stets immanenten Gefahr eines Verwechselns bzw. Verlorengehens) und zuverlässiger (da dank der Raststufen eine schnelle und dennoch genaue Einstellung der unterschiedlichen Höhen gewährleistet ist).

Es ergibt sich noch ein weiterer Vorteil, und zwar in Bezug auf eine bessere individuelle Adaption der Endoprothese an die anatomischen Verhältnisse des Patienten. Denn es kann mit dem aufgesetzten und gesicherten Probe-Halsstück ein Durchbewegen des Gelenks erfolgen (d. h. bspw. bei der Implantation einer Hüftgelenkendoprothese wird der Oberschenkel bewegt von der ausgestreckten in die angezogene Position und zurück). Auf diese Weise kann geprüft werden, ob ein ausreichender Bewegungsbereich ("Range of Motion") gewährleistet ist. Erforderlichenfalls kann eine andere Schaftlänge durch Verändern der Höhenstufe eingestellt werden. Damit verbessert sich nicht nur die Handhabung, sondern es kann auch insgesamt eine bessere Anpassung der Endoprothese intraoperativ erfolgen. Das Risiko einer fehlerhaften Implantation, was die Gefahr aufwendiger und den Patienten belastender Nach-Operationen mit sich bringt, kann damit wirksam minimiert werden.

Vorzugsweise ist eine Sicherungseinrichtung vorgesehen, welche ein Verrasten des Probe-Halsstücks in der jeweiligen Höhenstufe sichert. Damit wird eine einfache, schnelle und zuverlässige Sicherung der jeweils eingestellten Höhe erreicht, und zwar ohne dass gesonderte Teile wie Zwischenringe o. ä. benötigt würden. Mit Vorteil sind die Verrastungseinrichtung und/oder Sicherungseinrichtung selbstverriegelnd. Auf diese Weise sind gesonderte Handgriffe für das Verriegeln der Verrastungseinrichtung nicht erforderlich, sondern diese schnappen sozusagen selbsttätig ein. Damit ergibt sich eine bedeutend einfachere Handhabung. Auch die Gefahr einer versehentlich unterbliebenen Verriegelung der Verrastungseinrichtung wird auf diese Weise minimiert.

Zweckmäßigerweise ist an dem Probe-Halsstück eine Führung für eine lösbare Schnellkupplung mit einem Einsetzinstrument vorgesehen. Damit kann das Einsetzinstrument auf einfache und schnelle Weise sicher mit dem Probe-Halsstück verbunden werden. Insbesondere ist die Bindung sowohl zugfest wie auch winkelstabil, d. h. die relative Winkellage zwischen dem Probe-Halsstück und dem Einsetzinstrument ist fixiert.

Vorzugsweise weist hierbei die Führung eine winklige Führungsbahn auf mit einem Einführabschnitt und einem Arretierabschnitt, wobei von einem Anfang des Einführabschnitts gesehen der Arretierabschnitt als Hinterschnitt ausgeführt ist. Mit dem Arretierabschnitt wird das Einsetzen der Schnellkupplung vereinfacht, und mit der hinterschnittenen Ausführung des Arretierabschnitts wird eine besonders sichere und intrinsisch gegen ein unbeabsichtigtes Lösen geschützte Verbindung erreicht.

Mit Vorteil ist der Befestigungsbereich für ein Aufstecken auf Umschlag ausgeführt, vorzugsweise mit jeweils einer Verdrehung in 120°-Stufen oder weniger. Unter "auf Umschlag" wird verstanden, dass das Probe-Halsstück in verschiedenen Rotationspositionen bezogen auf eine Längsachse des Probe-Halsstücks aufgesetzt werden kann, wobei die einzelnen Rotationspositionen vorzugsweise durch die Außenkontur des oberen Bereichs des Schafts definiert sind.

Es ist bevorzugt, wenn der Befestigungsbereich an seinem unteren Ende eine Schulter aufweist, die eine Einstecktiefe begrenzt. Damit kann ein übermäßig weites Aufstecken des Probe-Halsstücks auf den Schaftkörper vermieden werden.

Zweckmäßigerweise ist das Probe-Halsstück so ausgebildet, dass es zur schraubenlosen Befestigung am Schaftkörper ausgebildet ist. Damit ergibt sich eine besonders einfache und schnelle Verbindung zwischen Probe-Halsstück und dem Schaftkörper.

Bevorzugt ist der Befestigungsbereich als Schiebehülse mit einer Durchgangsöffnung zum Aufstecken auf den Schaftkörper ausgeführt, die vorzugsweise kippbar ist. Das Aufstecken ermöglicht eine schnelle Montage, wobei die Verkippbarkeit dazu genutzt werden kann, das Verschieben des Probe-Halsstücks von einer Höhenstufe in eine andere zu vereinfachen. Insbesondere kann vorgesehen sein, dass ein Teil einer Innenwandung der Durchgangsöffnung schräg als Rampe ausgeführt ist. Mit dieser Ausführung wird auf besonders einfache Weise mittels der Rampe ein Freiraum bereitgestellt, der dem Schaft ein Verkippen innerhalb der Durchgangsöffnung ermöglicht.

Besonders zweckmäßig ist es, wenn an der Durchgangsöffnung eine Rastnase angeordnet ist, die in einer Riegelposition in eine Vertiefung des Schaftkörpers eingreift und in einer Verkippungsposition frei vom Schaftkörper ist, wobei die Rastnase vorzugsweise starr an der Durchgangsöffnung angeordnet ist. Damit ergibt sich eine formschlüssige Verbindung zwischen Probe-Halsstück einerseits und dem Schaftkörper andererseits, was einen hohen Schutz gegenüber unbeabsichtigter Verschiebung ermöglicht. Ferner ermöglicht dies in der Verkippungsposition eine leichte Verschiebbarkeit. Vorzugsweise ist die Rastnase gegenüberliegend von dem Halsbereich angeordnet. Damit kann erreicht werden, dass bei Belastung, also insbesondere bei Krafteinleitung auf den Halsbereich, die Rastnase in die Vertiefung gedrückt wird. Auf diese Weise wird eine selbstsichernde Wirkung erreicht, deren Wirksamkeit umso größer ist je höher die Belastungskraft ist. Bewährt hat es sich, wenn die Rastnase auf derselben Seite der Durchgangsöffnung angeordnet ist wie die Rampe. Insbesondere ist die Rastnase zweckmäßigerweise im Bereich des Anfangs der Rampe in der Durchgangsöffnung angeordnet. Damit kann eine besonders günstige Gestaltung des Übergangsbereichs zur Rampe hin erreicht werden.

Mit Vorteil weist das Probe-Halsstück eine Andruckfeder, vorzugsweise eine Blattfeder, auf, die auf den eingesteckten Schaftkörper wirkt. Diese ist dazu ausgebildet, das Probe-Halsstück in die Riegelposition zu drücken, so dass somit eine einfache und sichere Verrastung der jeweiligen Höhenstufe erreicht wird. Zweckmäßigerweise ist ein Sperrelement für die Andruckfeder vorgesehen. Dieses blockiert eine Bewegung der Andruckfeder, so dass diese nicht ausweichen kann und somit das Probe-Halsstück in der Riegelposition verzwängt wird.

Bei einer bevorzugten Ausführungsform ist ein Sichtfenster vorgesehen, welches so angeordnet ist, dass es bei eingesetztem Schaftkörper ein der jeweiligen Rastposition zugeordnetes Kennzeichnungsfeld zeigt. Mit Vorteil ist das Kennzeichnungsfeld mit einem Marker für ein Größen- und/oder Tiefenmaß versehen. Somit kann durch einfache Sichtkontrolle anhand des im Sichtfenster erscheinenden Markers verifiziert werden, ob eine gewünschte Einstellung erreicht ist. Der Gefahr von Fehleinstellungen wird damit auf so einfache wie effektive Weise entgegengewirkt. Zweckmäßigerweise ist hierbei vorgesehen, dass das Kennzeichnungsfeld mit einem Marker für ein Größen- und/oder Tiefenmaß versehen ist.

Vorzugsweise ist der gesonderte Schaftkörper als ein Räumwerkzeug, insbesondere als Reibahle, ausgeführt. Wie bereits einleitend angemerkt, eignet sich eine Reibahle (oder ein anderes Räumwerkzeug) in besonders guter Weise als Schaftkörper für das erfindungsgemäße Probe-Halsstück.

Die Erfindung erstreckt sich auch auf eine Anordnung umfassend das Probe-Halsstück und ein solches Räumwerkzeug, insbesondere Reibahle. Zweckmäßigerweise ist am oberen Ende des Räumwerkzeugs ein Standardanschluss für chirurgische Instrumente ausgebildet, vorzugsweise als ein Hudson-Anschluss. Außerdem kann zusätzlich oder alternativ das Räumwerkzeug, insbesondere Reibahle, zweiteilig mit einem proximalen und einem distalen Teil ausgeführt sein, die beide unverlierbar winkelgelenkig und drehfest verbunden sind. Damit ergibt sich eine für die chirurgische Praxis besonders günstige Gestaltung des Räumwerkzeugs, insbesondere der Reibahle.

Weiter erstreckt sich die Erfindung auf ein Instrumentarium umfassend ein Probe-Halsstück wie vorstehend erläutert sowie ein Einsetzinstrument, das an seinem distalen Ende mit einer lösbaren Schnellkupplung zur winkelfesten Aufnahme des Probe-Halsstücks versehen ist. Dabei weist das Einsetzinstrument vorzugsweise an seinem vorderen Ende ein Greifmodul mit einer starren Aufnahmegabel und einem am Grund der Aufnahmegabel längsverschieblich angeordneten Spannkörper auf, der vorzugsweise ebenfalls gabelförmig ausgebildet ist. Mit einer solchen Kombination aus Gabel und Spannkörper kann das Probe-Halsstück auf besonders gut zu handhabende Weise sicher und einfach ergriffen sowie zuverlässig gespannt und gegen unerwünschte Bewegung gesichert werden.

Mit Vorteil ist der Spannkörper mittels eines Schiebeorgans betätigt, das vorzugsweise Teil einer Fixierungseinrichtung ist. Hierbei ist die Fixierungseinrichtung vorzugsweise bistabil ausgeführt, vorzugsweise mittels eines Übertotpunktmechanismus, weiter vorzugsweise mit zwei Anschlägen für Greif- und Löseposition. Mit der Übertotpunktmechanik kann die Fixiersicherungseinrichtung leicht zwischen der geöffneten und der geschlossenen (fixierten) Position umgeschaltet werden. Es gibt nur zwei stabile Stellungen, was der Gefahr von Fehlbedingungen entgegenwirkt. Es ergeben sich somit beträchtliche Handhabungsvorteile für das Instrument.

Zweckmäßigerweise ist das Schiebeorgan als Federstab ausgeführt. Damit können zum einen Spannkräfte ausgeübt werden auf den Spannkörper und zum anderen ergibt sich ein automatischer Spielausgleich, so dass das Probe-Halsstück auch bei unvermeidlich auftretenden Toleranzen hinsichtlich seiner Abmessungen sicher gehaltert werden kann.

Gemäß einer weiteren bevorzugten Ausführungsform erstreckt sich die Erfindung auch auf ein Instrumentarium, das ein Adapterstück umfasst, welches nachfolgend zum Probe-Halsstück auf den Schaftkörper aufsetzbar und mit diesem verrastbar ist. Mithilfe dieses Adapterstücks kann der Schaftkörper, insbesondere die Reibahle, auf einfache Weise aus dem Markkanal extrahiert werden. Es wird hierbei vorzugsweise der gleiche Rastmechanismus verwendet wie bei der Verrastungseinrichtung des Probe-Halsstücks.

Nachfolgend wird die Erfindung anhand der Zeichnung beispielhaft anhand von vorteilhaften Ausführungsformen erläutert. Es zeigen:
- Fig. 1: eine Übersichtsdarstellung über ein Instrumentarium zum Räumen und Proben;
- Fig. 2a, b: Detaildarstellungen einer Reibahle gemäß einer Ausführungsform der Erfindung;
- Fig. 3a-c: Darstellungen zu einem Probe-Halsstück gemäß einer Ausführungsform;
- Fig. 4a-d: Probe-Halsstück in verschiedenen Positionen mit und ohne Verrastung bzw. Sicherung;
- Fig. 5a, b: eine Aufsicht auf das Probe-Halsstück darstellend eine Übergangs- und eine verrastete Positionierung;
- Fig. 6a-c: mehrere Ansichten eines Einsetzinstruments für das Probe-Halsstück;
- Fig. 7a, b: Ansichten eines Greifers des Einsetzinstruments, im offenen und verriegelten Zustand;
- Fig. 8a-c: Darstellungen zu verschiedenen Phasen beim Ankoppeln und des Probe-Halsstücks an das Einsetzinstrument; und

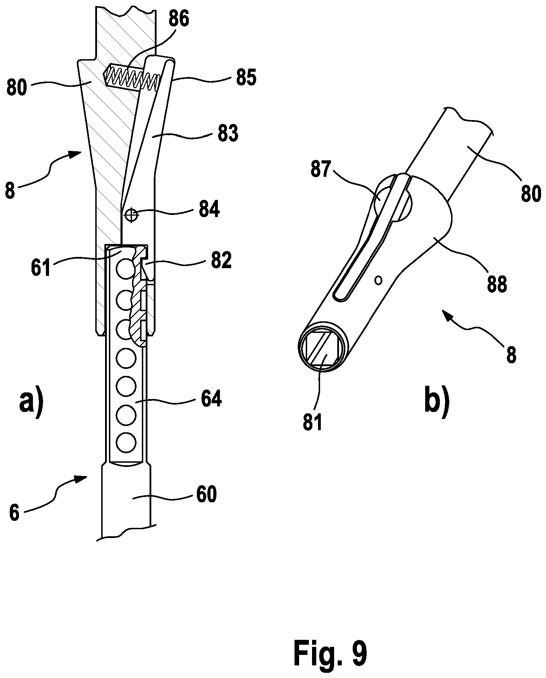

- Fig. 9a, b: Ansichten zu einem Extraktionsadapter für die Reibahle.

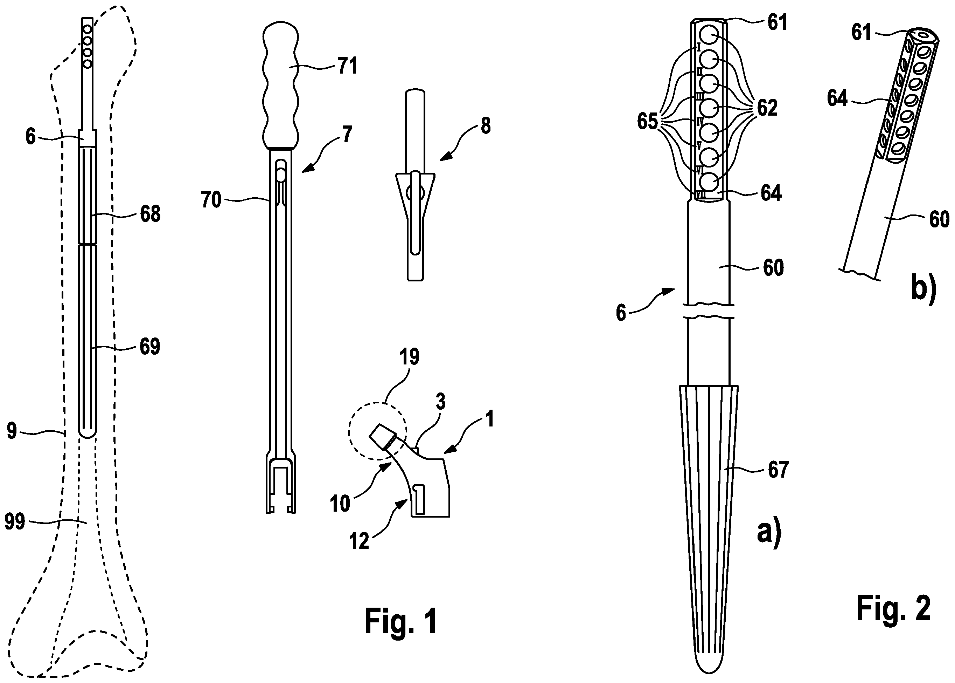

Eine beispielhafte Ausführungsform für ein Instrumentarium mit einem Probe-Halsstück gemäß einem Ausführungsbeispiel der Erfindung wird nachfolgend erläutert. Eine Übersicht über solch ein Instrumentarium ist in Figur 1 dargestellt. Es handelt sich um ein Instrumentarium zum Einsetzen einer Femurkomponente einer Hüftgelenkendoprothese (nicht dargestellt) in das proximale Ende eines Femurs 9. Hierbei weist die Femurkomponente einen langgestreckten Schaft zur Verankerung in einem Hohlraum des Femurs 9 auf. Zu diesem Zweck wird mittels des Instrumentariums ein natürlicherweise vorhandener Markkanal 99 im Femur erweitert zur Aufnahme des Schafts der Femurkomponente.

Die dargestellte Ausführungsform für ein erfindungsgemäßes Instrumentarium umfasst ein Probe-Halsstück 1, eine Reibahle 6, ein Einsetzinstrument 7 für das Probe-Halsstück 1 sowie einen Extraktionsadapter 8. Weitere Instrumente können vorgesehen sein, insbesondere solche, die üblicherweise zu einem Instrumentarium zum Implantieren einer Hüftgelenkendoprothese, und insbesondere von deren Femurkomponente, gehören.

Die Reibahle 6 ist dazu ausgebildet, den Markkanal 99 im Femur 9 an seinem proximalen und angrenzenden medialen diaphysären Bereich zu erweitern. Dies geschieht durch Räumen, wobei mittels eines Schneidbereichs 67 der Reibahle 6 Knochenmaterial aus dem Inneren des Femurs 9 abgetragen wird. Optional können vor der Reibahle 6 noch andere Instrumente zum Einsatz kommen, wie eine Knochensäge zum Entfernen eines defekten Schenkelhalses des Femurs 9 sowie Bohrer oder andere geeignete Werkzeuge zum Öffnen eines Zugangs zum Markkanal 99 des Femurs 9.

Das Räumen an sich kann in einen oder mehreren Zügen erfolgen, wobei gegebenenfalls auch progressiv größer werdende verschiedene Reibahlen (nicht dargestellt) zum Einsatz kommen. Zum Abschluss des eigentlichen Räumens ist zu prüfen, ob der Markkanal 99 ausreichend erweitert wurde. Dieses sog. Proben erfolgt konventionell häufig unter Verwendung eines gesonderten Probe-Implantats. Dies ist erfindungsgemäß nicht mehr erforderlich. Stattdessen wird erfindungsgemäß die zum Räumen verwendete und ohnehin in dem Markkanal 99 steckende Reibahle 1 verwendet. Dazu kann die Reibahle 1 an ihrer Position verbleiben; sie fungiert nunmehr mit ihrem Schaftkörper 60 als Schaft eines Probe-Implantats.

Das Probe-Halsstück 1 bildet im Wesentlichen den Halsbereich eines Probe-Implantats ab. Anders ausgedrückt entspricht es im Wesentlichen einem Probe-Implantat ohne dessen Schaft. Das Probe-Halsstück 1 weist einen Befestigungsbereich 12 auf, der zum Anordnen des Probe-Halsstücks 1 an dem Schaftkörper 60 ausgebildet ist, und einen Halsbereich 10, der wie ein herkömmlicher Halsbereich eines Probe-Implantats bzw. Implantats ausgebildet ist. Der Halsbereich 10 ist dazu ausgebildet, ein Gelenkelement (Gelenkkugel 19) der Gelenkendoprothese zu tragen. Zum Tragen der Gelenkkugel 19 ist an dem Halsbereich 10 an dessen äußeren Ende vorzugsweise ein Aufnahmekonus 18 in an sich bekannter Weise ausgebildet. Es kann so mit gleicher Geometrie wie bei der endgültigen Gelenkendoprothese die Gelenkkugel 19 montiert werden, und so das Gelenk unter Nutzung der Gelenkendoprothese bewegt werden, um so den richtigen Sitz der Prothese und Beweglichkeit der Gliedmaßen zu prüfen. Dieser Vorgang wird auch als "Proben" bezeichnet. Stellt sich hierbei heraus, dass der Sitz nicht optimal ist, beispielsweise weil der Freiraum im Markkanal 99 noch nicht hinreichend tief ausgearbeitet wurde, so kann dies dann durch weiteres Räumen des Markkanals 99 korrigiert und leicht überprüft werden, ohne dass dazu die Reibahle 6 entfernt zu werden braucht oder gar die endgültige Gelenkendoprothese dazu herangezogen werden müsste.

Zum Proben wird das erfindungsgemäße Probe-Halsstück 1 aufgesetzt auf den Schaftkörper 60 der Reibahle 6. Das Probe-Halsstück 1 weist dazu einen als Schiebehülse 13 ausgebildeten Hauptkörper mit einer Durchgangsöffnung 14 auf. Das Probe-Halsstück 1 wird mit seiner Durchgangsöffnung 14 an einem Kopfteil 61 des Schaftkörpers 60 der Reibahle 6 aufgesteckt. Dieser Kopfteil 61 des Schaftkörpers ist als Vierkant 64 ausgebildet. Der Vierkant 64 fungiert als Antriebskopf der Reibahle 6. Damit ist das Probe-Halsstück 1 formschlüssig an dem Schaftkörper 60 befestigt, so dass keine relative Verdrehung zwischen Probe-Halsstück 1 und dem Schaftkörper 60 auftreten kann. Wie weit das Probe-Halsstück 1 auf den Schaftkörper 60 der Reibahle 6 aufgesteckt werden kann, ist variabel. Es sind mehrere Höhenstufen dazu vorgesehen, so dass die Aufstecktiefe des Probe-Halsstücks 1 auf den Schaftkörper 60 variiert werden kann. Auf diese Weise können je nach gewählter Höhenstufe unterschiedliche Längen von Probe-Implantaten simuliert werden. Zur Begrenzung der Einstecktiefe ist eine Schulter 17 am unteren Ende des Befestigungsbereichs 12 des Probe-Halsstücks 1 vorgesehen.

Für die verschiedenen Höhenstufen sind mehrere Rastlöcher 62 in einer Reihe entlang der Achse des Schaftkörpers 60 oben am Kopfteil 61 an mindestens einer Seite des Vierkants 64 angeordnet. Vorzugsweise befindet sich die Anordnung der Rastlöcher 62 an mehreren Seiten des Vierkants 64, um so ein Umsetzen des Probe-Halsstücks 1 bezüglich seiner Winkelposition (in 90°-Schritten bei einem Vierkant) zu ermöglichen ("auf Umschlag"). An einer Seite des Vierkants 64 sind ferner den Rastlöchern 62 zugeordnete Kennzeichnungsfelder 65 vorgesehen. Diese tragen vorzugsweise Marker für die verschiedenen Raststufen 20, im dargestellten Beispiel I-VII für die durch die sieben Rastlöcher 62 definierten sieben Höhenstufen. An dem Probe-Halsstück 1 ist ein korrespondierendes Sichtfenster 21 vorgesehen. Es ist als Ausschnitt ausgeführt und ermöglicht von außen einen Blick auf diejenige Seitenfläche des Vierkants 64, die mit den Kennzeichnungsfeldern 65 versehen ist. Im Sichtfenster 21 erscheint somit das zu dem jeweiligen Rastloch 62 korrespondierende Kennzeichnungsfeld 65 mit seiner Markierung, so dass auf diese Weise einfach sowie verwechslungssicher die eingestellte Höhenstufe abgelesen werden kann.

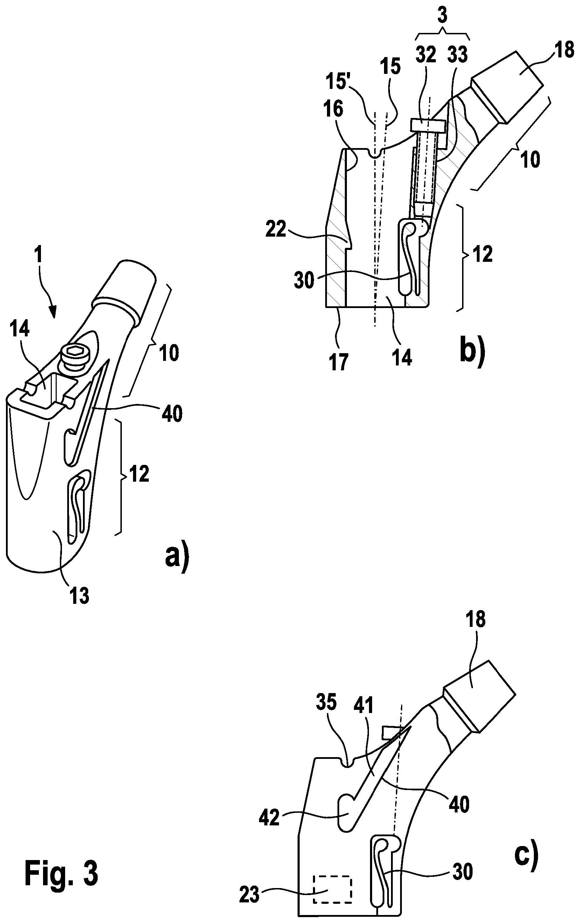

Zum Zusammenwirken mit den Rastlöchern 62 ist an einer Innenseite der Durchgangsöffnung 14 eine Rastnase 22 vorgesehen. In dem dargestellten Ausführungsbeispiel ist die Rastnase 22 in der Durchgangsöffnung 14 an der dem Halsbereich 10 gegenüberliegenden Seitenfläche angeordnet. Die Rastnase 22 ist sägezahnartig ausgeführt mit einem geneigten oberen Bereich und einem steilen unteren Bereich (siehe Figur 3 b). Der schräge obere Bereich erleichtert das Einrasten der Rastnase 22 in eines der Rastlöcher 62, während der steile untere Bereich im eingerasteten Zustand wie eine Anschlagschulter wirkt, und so eine hohe und sichere Kraftübertragung zwischen Probe-Halsstück 1 und dem Schaftkörper 60 der Reibahle 6 ermöglicht. Dank dieser steilen Ausführung der Anschlagschulter wird erreicht, dass auch bei hoher Krafteinwirkung keine Gefahr eines unerwünschten Herausspringens der Rastnase 22 aus dem Rastloch 62 besteht. Gemeinsam bilden so die Rastnase 22 mit jeweils einem der Rastlöcher 62 eine Raststufe 20 einer Verrastungseinrichtung 2. Die Anzahl der Raststufen 20 entspricht bei dem dargestellten Ausführungsbeispiel der Anzahl der Rastlöcher 62. Somit kann durch Verschieben des Probe-Halsstücks 1 auf dem Vierkant 64 und Einrasten in eines der Rastlöcher 62 eine definierte Höhe des Probe-Halsstücks 1 relativ zu dem Schaftkörper 60 der Reibahle eingestellt und sicher arretiert werden. Auf diese Weise können verschiedene Größen von Implantaten somit unter Verwendung von ein und derselben Reibahle 6 bzw. Probe-Halsstück 1 nachgebildet werden.

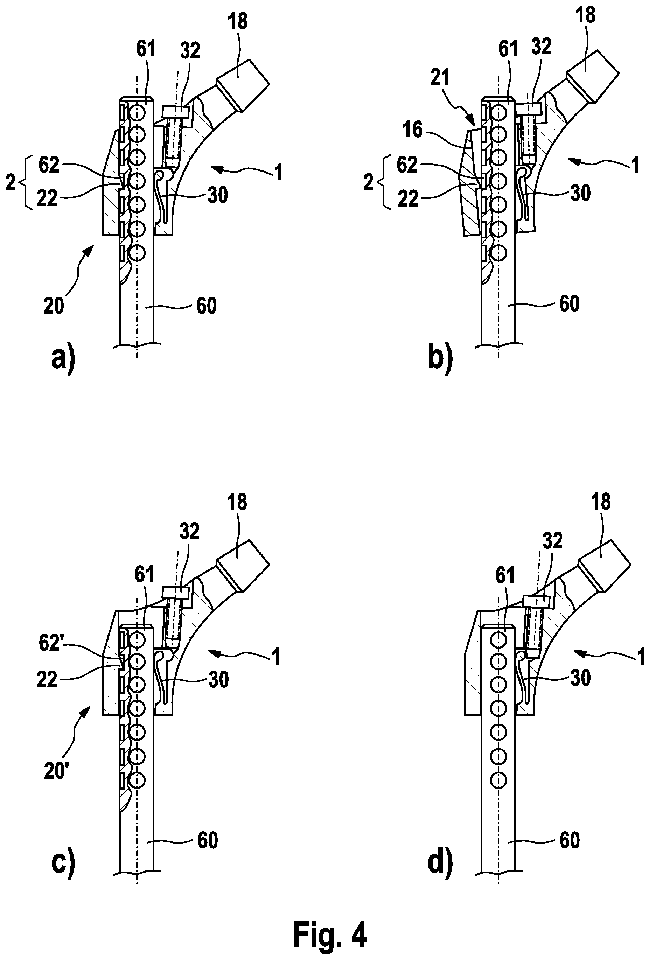

Um ein schnelles und sicheres Einrasten der Rastnase 22 in das jeweilige Rastloch 62 zu fördern, ist in der Durchgangsöffnung 14 ferner eine als Blattfeder ausgeführte Andruckfeder 30 vorgesehen. Sie ist an der Innenseite der Durchgangsöffnung 14 angeordnet, und zwar an derjenigen Innenseite, die der Rastnase 22 gegenüberliegt. Damit wirkt die Andruckfeder 30 auf die gegenüberliegende Fläche des Vierkants 64, und drückt diesen somit insgesamt in Richtung der Rastnase 22. Ein sicheres Einrasten zwischen Rastnase 22 und dem entsprechenden Rastloch 62 wird damit begünstigt. Auf diese Weise ergibt sich für die Verrastungseinrichtung 2 ein nahezu selbsttätiges Einrasten, sobald das Probe-Halsstück 1 entlang des Schaftkörpers 60 in die passende Position geschoben wurde. Dieser eingerastete Zustand ist in den Figuren 4 a und 4 c visualisiert.

Der Verschiebevorgang zwischen verschiedenen Rastlöchern 62 ist in Figur 4 b visualisiert. Man erkennt, dass die Rastnase 22 nicht in eines der Rastlöcher 62 eingreift, sondern vielmehr auf der Außenfläche des Vierkants 64 aufsitzt (bzw. bei Bewegung entlang gleitet). Man erkennt weiter, dass das Probe-Halsstück 1 hierbei gegenüber der eingerasteten Position (vergleiche Figur 4 a und 4 c) verkippt positioniert ist. Diese Verkippung ermöglicht es, dass sich die Rastnase 22 außerhalb der Rastlöcher 62 befindet und somit das Probe-Halsstück 1 leicht entlang dem Vierkant 64 des Schaftkörpers 60 verschoben werden kann. Um dies zu erleichtern, ist in der Durchgangsöffnung 14 auf derselben Seite, auf der auch die Rastnase 22 angeordnet ist, eine als schräge Rampe 16 ausgeführte Ausnehmung vorgesehen. Wie insbesondere gut in Figur 3 b zu erkennen, ist an dieser Stelle die Wandung der Durchgangsöffnung 14 nicht parallel zu der gegenüberliegenden Seite ausgeführt, sondern um einen bestimmten Winkel verkippt. Man erkennt dies auch an der gestrichelten Linie, wobei die durchgehende vertikale Linie die Mittelachse 15 der Durchgangsöffnung 14 darstellt, und die sich ergebende Verkippung infolge der Rampe 16 durch die links davon angeordnete, um einige Grad verkippte Linie 15' visualisiert ist. Um den sich daraus ergebenden Winkelbetrag kann das Probe-Halsstück 1 beim Verschieben von einem Rastloch 62 zu einem anderen der Rastlöcher 62 verkippt werden, wie in Figur 4 b dargestellt ist. Ist das gewünschte Rastloch 62' erreicht, so wird das Probe-Halsstück 1 unter der Wirkung der Andruckfeder 30 wieder in die ursprüngliche Position, d. h. parallel ausgerichtet zur Mittelachse 15, zurückgekippt, wobei die Rastnase 22 in das Rastloch 62' eingreift. Damit ist die Verrastungseinrichtung 2 in einer anderen Raststufe 20' verriegelt gegenüber einer unerwünschten Längsbewegung des Probe-Halsstücks 1 entlang des Schaftkörpers 60.

Um einen zusätzlichen Schutz der Verrastungseinrichtung 2 gegenüber einer unerwünschten Bewegung bzw. Betätigung zu erreichen, ist zusätzlich eine Sicherungseinrichtung 3 vorgesehen. In dem dargestellten Ausführungsbeispiel ist sie ausgeführt als eine Sicherungsschraube 32 in einer Nebenbohrung 33. Die Sicherungsschraube 32 ist im Übergang zwischen dem Halsbereich 10 und dem Befestigungsbereich 12 des Probe-Halsstücks 1 angeordnet, und die Nebenbohrung 33 ist vorzugsweise zur Durchgangsöffnung 14 benachbart und etwa parallel angeordnet, wobei die Nebenbohrung 33 im Bereich der Andruckfeder 30 endet. Durch Einschrauben der Sicherungsschraube 32 wird deren Schaft so weit nach unten bewegt, dass zumindest die Spitze des Schafts der Sicherungsschraube 32 die Andruckfeder 30 gegen die Seitenflächen des Vierkants 64 des Schaftkörpers 60 drückt, und somit den Schaftkörper 60 in seiner unverkippten Lage in der Durchgangsöffnung 14 des Probe-Halsstücks 1 verzwängt. Eine Verkippung ist somit nicht mehr möglich, so dass die Rastnase 22 in dem entsprechenden Rastloch 62 gefangen und eine Längsbewegung der Verrastungseinrichtung 2 zuverlässig gesperrt ist. Durch Herausdrehen der Sicherungsschraube 32 kann diese Sicherung auf Wunsch wieder gelöst werden.

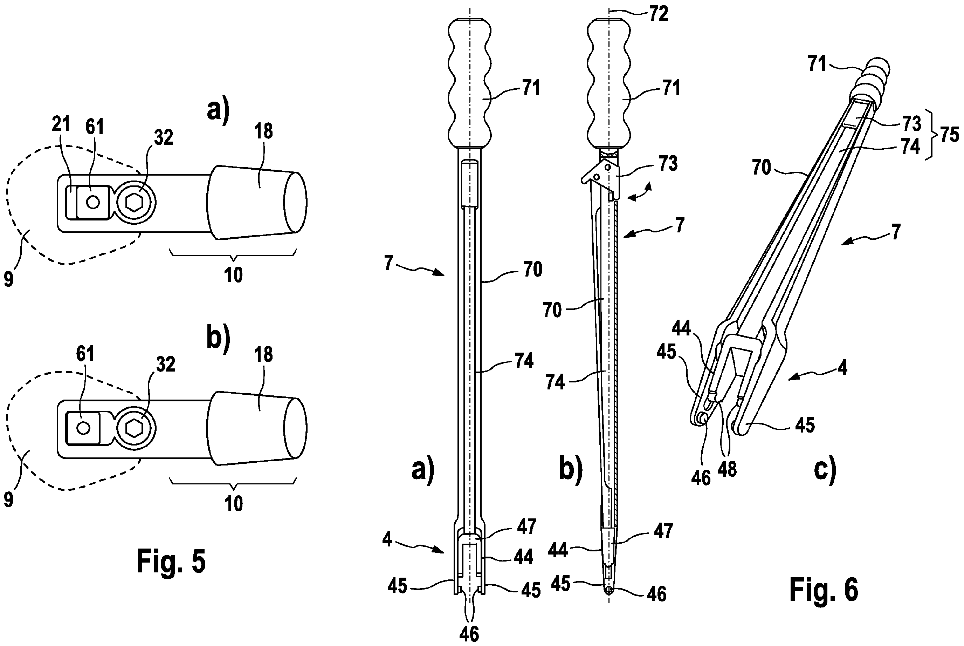

Um dem Chirurgen eine einfache und auch von außerhalb gut sichtbare Kontrolle zu ermöglichen, ob die Verrastungseinrichtung 2 sicher eingerastet ist, ist ein Prüffenster 21 vorgesehen. Dieses ist am oberen Ende des Probe-Halsstücks 1 gebildet (siehe Figur 5 a), und zwar zwischen dem oberen Ende 61 des Schaftkörpers 60 und der von dem Halsbereich 10 abgewandten Seite des Probe-Halsstücks 1. Das Prüffenster 21 ist sichtbar im verkippten Zustand des Probe-Halsstücks 1, wie auch in Figur 4 b visualisiert ist. Sowie dieses Prüffenster 21 sichtbar ist, ist die Verrastungseinrichtung 2 nicht eingerastet und es liegt somit ein unsicherer Zustand vor. Erst dann, wenn das Probe-Halsstück 1 wieder seinen unverkippten Zustand eingenommen hat (siehe Figur 5 b), verschwindet das Prüffenster 21. Dies ist das Zeichen dafür, dass nunmehr die Verrastungseinrichtung 2 wieder eingerastet ist. Nunmehr kann gewünschtenfalls zur weiteren Sicherung die Sicherungsschraube 32 eingedreht werden, um so den gesicherten Zustand gemäß Figur 4 d zu erreichen.

Zum Einsetzen des Probe-Halsstücks 1 und gegebenenfalls Extraktion von dem Probe-Halsstück 1 mitsamt Reibahle 6 ist ein Einsetzinstrument 7 vorgesehen. Es ist ausgeführt mit einem langen Schaft 70, an dessen einem Ende ein Handgriff 71 angeordnet ist. Am gegenüberliegenden Ende des Schafts 70 ist eine Schnellkupplung 4 vorgesehen, die ein Greifmodul 44 umfasst. Das Greifmodul 44 umfasst eine Aufnahmegabel 45, an deren einander zugewandten Innenflächen jeweils ein Haltezapfen 46 angeordnet ist. Die Haltezapfen 46 sind in dem dargestellten Ausführungsbeispiel als zylinderartige Vorsprünge ausgebildet. Das Greifmodul 44 umfasst ferner einen Spannschlitten 47, der in Längsrichtung des Schafts verschieblich zwischen den Innenflächen der Aufnahmegabel 45 angeordnet ist. Der Spannschlitten 47 ist an seinem schaftfernen Ende gabelartig geformt, wobei an der Spitze Drucknasen 48 vorgesehen sind. Schaftseitig ist der Spannschlitten 47 angeordnet an einem als Schiebestift ausgeführten Schiebeorgan 74, der längsverschieblich entlang einer Mittelachse 72 des Schafts 70 des Einsetzinstruments 7 geführt ist. Das Schiebeorgan 74 ist vorzugsweise als Federstab ausgeführt.

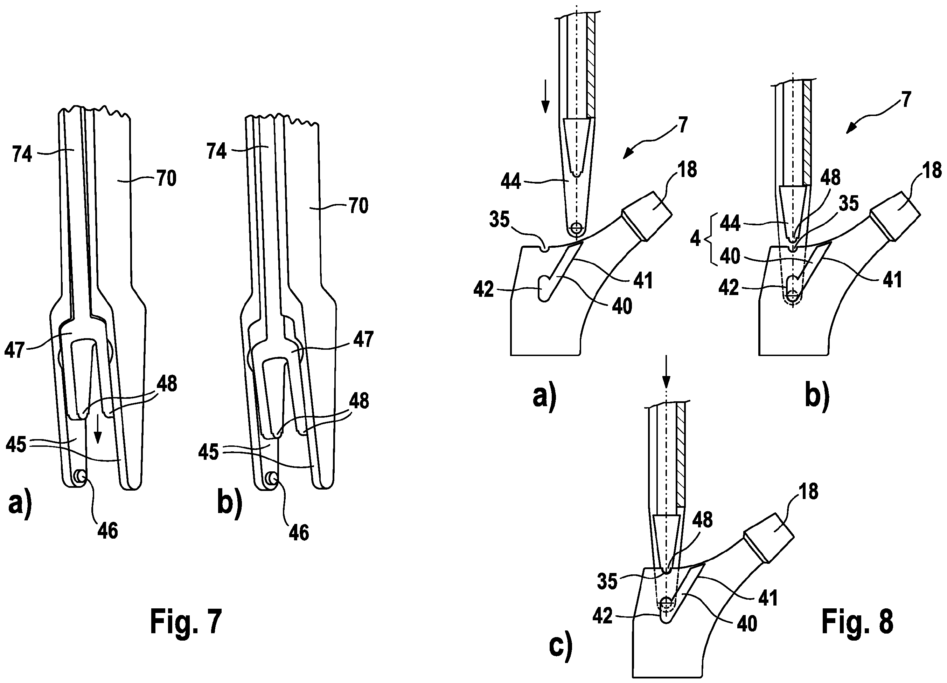

Das Greifmodul 44 ist dazu ausgebildet, mit einer Führungsbahn 40 an dem Probe-Halsstück 1 zusammenzuwirken. Die Führungsbahn 40 ist gewinkelt ausgeführt mit einem langgestreckten sich vom oberen Rand aus schräg zur Richtung der Mittelachse 15 erstreckenden Einführabschnitt und einem sich an dessen fernen Ende anschließenden Arretierabschnitt 42, der sich hinterschnittartig in Richtung des oberen Rands des Probe-Halsstücks 1 erstreckt. Die Führungsbahn 40 ist zur Aufnahme der Haltezapfen 46 bemessen. Diese können entlang des Einführabschnitts 41 auf den Hauptkörper des Probe-Halsstücks 1 aufgeschoben werden (s. Fig. 8 a), um schließlich in dem hinterschnittenen Arretierabschnitt 42 eine Halteposition einzunehmen (s. Fig. 8 b). Anschließend können die Drucknasen 48 zur Anlage gebracht werden an die Oberseite des Probe-Halsstücks 1. Dazu ist vorzugsweise ein Drucksattel 35 vorgesehen mit einer an die Außenkontur der Drucknasen 48 angepassten Konkavität. Damit kann durch Verspannen des Spannschlittens 47 mit seinen Drucknasen 48 gegenüber den Haltezapfen 46 das Probe-Halsstück 1 sicher an dem Einsetzinstrument 7 gehaltert werden, und zwar sicher in Bezug auf eine feste Arretierung sowohl in Längsrichtung wie auch gegenüber Verkippungen (s. Fig. 8 c).

Das als Federstab ausgeführte Schiebeorgan 74 bildet zusammen mit einem Schwenkelement 73 einen Übertotpunktmechanismus (s. Fig. 6). Dieser stellt eine bistabile Fixierungseinrichtung für das Greifmodul 44 dar. Das Schwenkelement 73 wirkt mit seiner Vorderseite auf das Schiebeorgan 74 und bewegt dieses abhängig von einer Schwenkposition vorwärts. Dadurch wird entsprechend der am vorderen Ende des Schiebeorgans 74 angeordnete Spannschlitten 47 vorgeschoben (s. auch den Pfeil in Fig. 7 a). Das Schwenkelement 73 weist zwei Anschläge auf, die jeweils eine Endposition definieren. In der einen Endposition ist das Schiebeorgan 74 mit dem Spannschlitten 47 zurückgezogen und in der anderen Endposition befindet sich das Schiebeorgan 74 mit dem Spannschlitten 47 in vorgeschobener Position. Das Verschwenkelement 73 bildet so mit dem Schiebeorgan 74 eine Übertotpunktmechanik, mit der der Spannschlitten 47 in der vorgeschobenen Position fixiert und, dank der Ausführung des Schiebeorgans 74 aus Federstahl, gespannt werden kann.

Das Betätigen des Verschwenkelements 73 und Spannen des Schiebeorgans 74 geschieht dann, wenn das Greifmodul 44 mit seinen Haltezapfen 46 in die Führungsbahn 40 am Probe-Halsstück 1 eingeführt und die Haltezapfen 46 in dem Arretierungsabschnitt 42 ihre Position eingenommen haben. Durch Verspannen wird dann der Spannschlitten 47 nach vorne bewegt, so dass dieser mit seinen Drucknasen 48 auf den Drucksattel 35 einwirkt, und das Probe-Halsstück 1 fest ergriffen und gehaltert wird. Es kann so sicher mittels des Einsetzinstruments 7 bewegt und positioniert werden. Diese Befestigung ist sicher und zu einer solch hohen Kraftübertragung befähigt, dass mittels des Einsetzinstruments 7 ggf. auch die an das Probe-Halsstück 1 über die Rasteinrichtung 2 gekoppelte Reibahle 6 zuverlässig aus dem Markkanal 99 des Knochens 9 extrahiert werden kann.

Das Instrumentarium kann ferner einen Extraktionsadapter 8 umfassen. Dieser ist dazu ausgebildet, um auf das obere Ende 61 des Schaftkörpers 60 der Reibahle 6 aufgesteckt zu werden. Der Extraktionsadapter 8 weist einen Schaft 80 auf, an dessen vorderem Ende ein Kopplungsstück angeordnet ist. Das Kopplungsstück umfasst eine konische Aufweitung 88. Am vorderen, freien Ende des Extraktionsadapters 8 ist eine quadratische Aufnahmeöffnung 81 vorgesehen. Sie ist dazu ausgebildet, das freie Ende 61 des Schaftkörpers 60 aufzunehmen. An einer Seite der Aufnahmeöffnung 81 ist eine Arretiereinrichtung 83 vorgesehen. Die Arretiereinrichtung 83 ist als Hebel ausgeführt, der über einen Lagerstift 84 schwenkbeweglich ist. An seinem fernen Ende weist der Hebel der Arretiereinrichtung 83 eine Haltenase 82 auf, welche in die Aufnahmeöffnung 81 hereinragt. Die Haltenase 82 ist dazu ausgebildet, mit einem der Rastlöcher 62 an dem Vierkant 64 des Schaftkörpers 60 der Reibahle 6 zusammenzuwirken, um so die Reibahle 6 an dem Extraktionsadapter 8 zugfest zu fixieren. In dem Bereich der Verdickung 88 ist eine Druckfeder 86 angeordnet, welche auf einen Betätigungshebel 85 der Arretiereinrichtung 83 wirkt. Sie ist so vorgespannt, dass sie den Betätigungshebel 85 in eine solche Position drückt, dass die Haltenase 82 in Richtung des Rastlochs 62 bewegt wird und somit verriegelt. Die Reibahle 6 ist somit zugfest an dem Extraktionsadapter 8 gehaltert und kann in an sich bekannter Weise aus dem Knochen 9 herausgezogen werden. Zum Lösen des Extraktionsadapters 8 braucht nur auf den Betätigungshebel 85 gedrückt zu werden, um die Haltenase 82 aus dem Bereich der Aufnahmeöffnung 81 heraus zu schwenken und somit die Reibahle 6 freizugeben. Für eine einfache Betätigung ist rund um den Betätigungshebel 85 eine Ausnehmung 87 in der konischen Aufweitung 88 als Fingeraufnahme vorgesehen.

Vorzugsweise weist der Extraktionsadapter 8 an seinem gegenüberliegenden Ende des Schafts 80 einen Adapter zum Anschluss an ein herkömmliches Instrumentarium auf, beispielsweise einen sogenannten Hudson-Adapter (nicht dargestellt). Damit können auch herkömmliche Werkzeuge zur sicheren Extraktion der Reibahle 6 verwendet werden. Es kann alternativ und/oder zusätzlich auch vorgesehen sein, einen solchen Hudson-Adapter am oberen Ende des Kopfteils 61 der Reibahle anzuordnen, sofern sein Querschnitt klein genug ist für einen Durchgang durch die Durchgangsöffnung 14 des Probe-Halsstücks 1.

## Patentansprüche

1. Probe-Halsstück für eine Gelenkendoprothese, das zur temporären Anordnung an einem in einen Röhrenknochen (9) steckbaren gesonderten Schaftkörper (60) ausgebildet ist, wobei das Probe-Halsstück (1) umfasst:
einen Befestigungsbereich (12), der mittels einer Steckverbindung auf ein Kopfteil (61) des Schaftkörpers (60) in mindestens einer definierten Position temporär aufsteckbar ist,
einen Halsbereich (10), der zur Aufnahme eines Gelenkelements (19) einer Gelenkendoprothese ausgebildet ist,
**dadurch gekennzeichnet, dass**
das Probe-Halsstück (1) als ein gesondertes, steckbares Aufsatzstück ausgeführt ist, das mit seinem Befestigungsbereich (12) formschlüssig auf das Kopfteil (61) aufsteckbar und arretierbar ist,
wobei eine Verrastungseinrichtung (2) vorgesehen ist, die mehrere, vorzugsweise mindestens fünf, Raststufen umfasst für verschiedene Höhenstufen des Probe-Halsstücks (1) am Kopfteil (61) des Schaftkörpers (60).

2. Probe-Halsstück nach Anspruch 1, **dadurch gekennzeichnet, dass** eine Sicherungseinrichtung (3) vorgesehen ist, welche ein Verrasten des Probe-Halsstücks (1) in der jeweiligen Höhenstufe am Kopfteil (61) des Schaftkörpers (60) sichert, wobei vorzugsweise die Verrastungseinrichtung (2) und/oder Sicherungseinrichtung (3) selbstverriegelnd ist.

3. Probe-Halsstück nach einem der vorangehenden Ansprüche, **dadurch gekennzeichnet, dass** an dem Probe-Halsstück (1) eine Führung für eine lösbare Schnellkupplung mit einem Einsetzinstrument vorgesehen ist und/oder das Probe-Halsstück zur schraubenlosen Befestigung am Schaftkörper (60) ausgebildet ist.

4. Probe-Halsstück nach Anspruch 3, **dadurch gekennzeichnet, dass** die Führung eine winklige Führungsbahn (40) aufweist, mit einem Einführabschnitt (41) und einem Arretierabschnitt (42), wobei von einem Anfang des Einführabschnitts (41) gesehen der Arretierabschnitt (42) als Hinterschnitt ausgeführt ist.

5. Probe-Halsstück nach einem der vorangehenden Ansprüche, **dadurch gekennzeichnet, dass** der Befestigungsbereich (12) für ein Aufstecken auf Umschlag ausgeführt ist, vorzugsweise mit jeweils einer Verdrehung in 120°-Stufen oder weniger, und/oder der Befestigungsbereich (12) an seinem unteren Ende eine Schulter (17) aufweist, die eine Einstecktiefe begrenzt.

6. Probe-Halsstück nach einem der vorangehenden Ansprüche, **dadurch gekennzeichnet, dass** der Befestigungsbereich (12) als Schiebehülse mit einer Durchgangsöffnung (14) zum Aufstecken auf den Schaftkörper (60) ausgeführt ist, die vorzugsweise kippbar ist, wobei vorzugsweise ein Teil einer Innenwandung der Durchgangsöffnung (14) schräg als Rampe (16) ausgeführt ist.

7. Probe-Halsstück nach Anspruch 6, **dadurch gekennzeichnet, dass** an der Durchgangsöffnung (14) eine Rastnase (22) angeordnet ist, die in einer Riegel-Position in eine Vertiefung (62) des Schaftkörpers (60) eingreift und in einer Verkippungsposition frei vom Schaftkörper (60) ist, wobei die Rastnase (22) vorzugsweise starr an der Durchgangsöffnung (14) angeordnet ist.

8. Probe-Halsstück nach Anspruch 7, **dadurch gekennzeichnet, dass** die Rastnase (22) gegenüberliegend von dem Halsbereich (10) angeordnet ist, so dass bei Krafteinleitung auf den Halsbereich (10) die Rastnase (22) in die Vertiefung (62) gedrückt wird, und/oder die Rastnase (22) auf derselben Seite der Durchgangsöffnung (14) angeordnet ist wie die Rampe (16).

9. Probe-Halsstück nach einem der vorangehenden Ansprüche, **dadurch gekennzeichnet, dass** es eine Andruckfeder (30), vorzugsweise eine Blattfeder, aufweist, die auf den eingesteckten Schaftkörper (60) wirkt und das Probe-Halsstück (1) in eine verrastete Position drückt, wobei vorzugsweise ein Sperrelement (32) für die Andruckfeder (30) vorgesehen ist.

10. Probe-Halsstück nach einem der vorangehenden Ansprüche, **dadurch gekennzeichnet, dass** ein Sichtfenster (23) vorgesehen ist, welches so angeordnet ist, dass es bei eingesetztem Schaftkörper (60) ein der jeweiligen Rastposition zugeordnetes Kennzeichnungsfeld (65) zeigt, wobei vorzugsweise das Kennzeichnungsfeld (65) mit einem Marker für ein Größen- und/oder Tiefenmaß versehen ist.

11. Probe-Halsstück nach einem der vorangehenden Ansprüche, **dadurch gekennzeichnet, dass** der gesonderte Schaftkörper (60) ausgeführt ist als ein Teil eines Räumwerkzeugs, insbesondere einer Reibahle (6), wobei vorzugsweise am oberen Ende des Räumwerkzeugs, insbesondere Reibahle (6), ein Standardanschluss für chirurgische Instrumente ausgebildet ist, vorzugsweise als ein Hudson-Anschluss, und/oder das Räumwerkzeug zweiteilig mit einem proximalen und einem distalen Teil (68, 69) ausgeführt ist, die unverlierbar winkelgelenkig und drehfest verbunden sind.

12. Instrumentarium umfassend ein Probe-Halsstück nach einem der vorangehenden Ansprüche sowie ein Einsetzinstrument (7), das an seinem distalen Ende mit einer lösbaren Schnellkupplung (4) zur winkelfesten Aufnahme des Probe-Halsstücks (1) versehen ist.

13. Instrumentarium nach Anspruch 12, **dadurch gekennzeichnet, dass** das Einsetzinstrument (7) an seinem vorderen Ende ein Greifmodul (44) mit einer starren Aufnahmegabel (45) und einem am Grund der Aufnahmegabel längsverschieblich angeordneten Spannkörper (47) aufweist, der vorzugsweise ebenfalls gabelförmig ausgebildet ist.

14. Instrumentarium nach Anspruch 13, **dadurch gekennzeichnet, dass** der Spannkörper mittels eines Schiebeorgans (48) betätigt ist, das vorzugsweise Teil einer Fixierungseinrichtung ist, und/oder das Schiebeorgan (48) als Federstab ausgeführt ist.

15. Instrumentarium nach Anspruch 14, **dadurch gekennzeichnet, dass** die Fixierungseinrichtung bistabil ausgeführt ist, vorzugsweise mittels eines Übertotpunkmechanismus (49), weiter vorzugsweise mit zwei Anschlägen für Greif- und Löseposition.

16. Instrumenten-Satz zum Implantieren einer Prothese in einen langgestreckten Knochen (Röhrenknochen) umfassend:
eine Reibahle, die dazu ausgebildet ist, von einem Ende des Röhrenknochens in einem Hohlraum des Knochens entlang einer langen Achse des Röhrenknochens eingesetzt zu werden und Knochenmaterial abzutragen, und
die an einem Kopfteil einen Antriebskopf aufweist, sowie
ein Probe-Halsstück nach einem der Ansprüche 1-11,
**dadurch gekennzeichnet, dass**
der Antriebskopf als ein Mehrkant ausgeführt ist,
das Probe-Halsstück als ein steckbarer Aufsatz ausgeführt ist, der mit seinem Befestigungsbereich formschlüssig auf den Antriebskopf aufsteckbar ist und
eine Verrastungseinrichtung vorgesehen ist, die mehrere Raststufen umfasst für eine Anordnung des Probe-Halsstücks in verschiedenen Höhen an der Reibahle, wobei das Probe-Halsstück in der Raststufe kippsicher arretiert ist,
und eine Sicherungseinrichtung vorgesehen ist, welche das Probe-Halsstück in der jeweiligen Raststufe fixiert.

## Claims

1. A trial neck piece for a joint endoprosthesis, which is designed for temporary arrangement on a separate shaft body (60) that is insertable into a tubular bone (9), wherein the trial neck piece (1) comprises:
a fastening region (12) which can be plugged temporarily onto a head part (61) of the shaft body (60) by means of a plug connection in at least one defined position,
a neck region (10), which is designed to receive a joint element (19) of a joint endoprosthesis, **characterized in that** the trial neck piece (1) is designed a as a separate, plug-on attachment piece which, with its fastening region (12) can be plugged with form-fit engagement onto the head part (61) and locked,
wherein a latching device (2) is provided, which comprises a plurality, preferably at least five latching stages for different height stages of the trial neck piece (1) on the head part (61) of the shaft body (60).

2. The trial neck piece according to claim 1, **characterized in that** a securing device (3) is provided, which secures a latching of the trial neck piece (1) in the respective height stage on the head part (61) of the shaft body (60), wherein preferably the latching device (2) and/or securing device (3) is self-locking.

3. The trial neck piece according to any of the foregoing claims, **characterized in that** a guide for a detachable quick coupling with an insertion instrument is provided on the trial neck piece (1) and/or the trial neck piece is designed for screwless fastening on the shaft body (60).

4. The trial neck piece according to claim 3, **characterized in that** the guide has an angular guide track (40) with an insertion section (41) and an arresting section (42), wherein viewed from a start of the insertion section (41) the arresting section (42) is designed as an undercut.

5. The trial neck piece according to any of the foregoing claims, **characterized in that** the fastening region (12) is designed for an attachment to the transition, preferably in each case with a rotation in 120° increments or less, and/or that the fastening region (12) has a shoulder (17) on its lower end, which limits an insertion depth.

6. The trial neck piece according to any of the foregoing claims, **characterized in that** the fastening region (12) is designed as a sliding sleeve with a through opening (14) for attachment on the shaft body (60), which is preferably tiltable, wherein preferably a portion of an inner wall of the through opening (14) is designed obliquely as a ramp (16).

7. The trial neck piece according to claim 6, **characterized in that** a latching lug (22) is arranged on the through opening (14), which in a locking position engages in a recess (62) of the shaft body (60) and in a tilting position is free from the shaft body (60), wherein the latching lug (22) is preferably arranged rigidly on the through opening (14).

8. The trial neck piece according to claim 7, **characterized in that** the latching lug (22) is arranged opposite the neck region (10), so that in the event of the application of force on the neck region (10) the latching lug (22) is pressed into the recess (62), and/or that the latching lug (22) is arranged on the same side of the through opening (14) as the ramp (16).

9. The trial neck piece according to any of the foregoing claims, **characterized in that** it has a pressure spring (30), preferably a leaf spring, which acts on the inserted shaft body (60) and presses the trial neck piece (1) to a latched position, wherein preferably a locking element (32) is provided for the pressure spring (30).

10. The trial neck piece according to any of the foregoing claims, **characterized in that** a viewing window (23) is provided which is arranged such that in the case of an inserted shaft body (60) it shows an identification field (65) assigned to the respective latching position, wherein preferably the identification field (65) is provided with a marker for a size and/or depth gauge.

11. The trial neck piece according to any of the foregoing claims, **characterized in that** the separate shaft body (60) is designed as part of an excavating tool, in particular a reamer (6), wherein preferably on the upper end of the excavating tool, in particular the reamer (6), a standard connection for surgical instruments is embodied, preferably as a Hudson connection, and/or that the excavating tool is designed in two parts with a proximal and a distal part (68, 69), which are non-removably and rotationally fixed at an angle.

12. An instrument set comprising a trial neck piece according to any of the foregoing claims as well as an insertion instrument (7), which is provide on its distal end with a detachable quick coupling (4) for receiving the trial neck piece (1) at a fixed angle.

13. The instrument set according to claim 12, **characterized in that** the insertion instrument (7) has a gripping module (44) with a rigid receiving fork (45) and a clamping body (47) arranged longitudinally displaceable at the base of the receiving fork, said clamping body being preferably likewise fork-shaped.

14. The instrument set according to claim 13, **characterized in that** the clamping body is actuated by means of a sliding element (48) which is preferably part of a fixing device, and/or that the sliding element (48) is designed as a spring bar.

15. The instrument set according to claim 14, **characterized in that** the fixing device is bistable, preferably by means of an over center mechanism (49), further preferably with two stops for grip and release positions.

16. An instrument set for implanting a prosthesis in an elongated bone (tubular bone) comprising: a reamer, which is designed to be inserted from an end of the tubular bone in a hollow space of the bone along a long axis of the tubular bone and to remove bone material, and which has a drive head on a head part, as well as a trial neck piece according to any of claims 1-11, **characterized in that** the drive head is designed as a polygon, the trial neck piece is designed as a plug-on attachment which, with its fastening region can be plugged with form-fit engagement onto the drive head and a latching device is provided, which comprises a plurality of latching stages for an arrangement of the trial neck piece in different heights on the reamer, wherein the trial neck piece is locked so that it cannot tilt in the latching stage, and that a securing device is provided, which fixes the trial neck piece in the respective latching stage.

## Revendications

1. Pièce de col d'essai pour une endoprothèse d'articulation, qui est réalisée pour être disposée temporairement sur un corps de tige (60) séparé pouvant être enfiché dans un os long (9), dans laquelle la pièce de col d'essai (1) comprend :
une zone de fixation (12), qui peut être enfichée temporairement au moyen d'une liaison d'enfichage sur une partie de tête (61) du corps de tige (60) dans au moins une position définie,
une zone de col (10), qui est réalisée pour recevoir un élément d'articulation (19) d'une endoprothèse d'articulation,
**caractérisée en ce que**
la pièce de col d'essai (1) est conçue en tant que pièce rapportée séparée pouvant être enfichée, qui peut être enfichée et peut être arrêtée par coopération de formes sur la partie de tête (61) avec sa zone de fixation (12),
dans laquelle un dispositif d'encliquetage (2) est prévu, qui comprend plusieurs, de préférence au moins cinq niveaux d'encliquetage pour différents niveaux de hauteur de la pièce de col d'essai (1) sur la partie de tête (61) du corps de tige (60) .

2. Pièce de col d'essai selon la revendication 1, **caractérisée en ce qu'**un dispositif de blocage (3) est prévu, lequel bloque un encliquetage de la pièce de col d'essai (1) dans le niveau de hauteur respectif sur la partie de tête (61) du corps de tige (60), dans laquelle de préférence le dispositif d'encliquetage (2) et/ou dispositif de blocage (3) est à verrouillage automatique.

3. Pièce de col d'essai selon l'une quelconque des revendications précédentes, **caractérisée en ce qu'**un guidage pour un accouplement rapide libérable avec un instrument d'insertion est prévu sur la pièce de col d'essai (1) et/ou que la pièce de col d'essai est réalisée pour la fixation sans vis sur le corps de tige (60).

4. Pièce de col d'essai selon la revendication 3, **caractérisée en ce que** le guidage présente une bande de guidage (40) angulaire, avec une section d'introduction (41) et une section d'arrêt (42), dans laquelle, vue depuis un début de la section d'introduction (41), la section d'arrêt (42) est conçue sous la forme d'un dégagement.

5. Pièce de col d'essai selon l'une quelconque des revendications précédentes, **caractérisée en ce que** la zone de fixation (12) est conçue pour un enfichage après retournement, de préférence avec respectivement une rotation par échelons de 120° ou moins, et/ou la zone de fixation (12) présente à son extrémité inférieure un épaulement (17), qui délimite une profondeur d'enfichage.

6. Pièce de col d'essai selon l'une quelconque des revendications précédentes, **caractérisée en ce que** la zone de fixation (12) est conçue sous la forme d'un manchon coulissant avec une ouverture de passage (14) pour l'enfichage sur le corps de tige (60), qui est de préférence basculant, dans laquelle de préférence une partie d'une paroi intérieure de l'ouverture de passage (14) est conçue de manière inclinée sous la forme d'une rampe (16).

7. Pièce de col d'essai selon la revendication 6, **caractérisée en ce qu'**un bec d'encliquetage (22), qui s'insère dans une position de verrou dans un évidement (62) du corps de tige (60) et est dégagé du corps de tige (60) dans une position de basculement, est disposé sur l'ouverture de passage (14), dans laquelle le bec d'encliquetage (22) est disposé de préférence de manière fixe sur l'ouverture de passage (14).

8. Pièce de col d'essai selon la revendication 7, **caractérisée en ce que** le bec d'encliquetage (22) est disposé à l'opposé de la zone de col (10), de sorte que lors de l'application de force sur la zone de col (10), le bec d'encliquetage (22) est pressé dans l'évidement (62), et/ou le bec d'encliquetage (22) est disposé sur la même face de l'ouverture de passage (14) que la rampe (16).

9. Pièce de col d'essai selon l'une quelconque des revendications précédentes, **caractérisée en ce qu'**elle présente un ressort de compression (30), de préférence un ressort à lames, qui agit sur le corps de tige (60) enfiché et presse la pièce de col d'essai (1) dans une position encliquetée, dans laquelle de préférence un élément de blocage (32) est prévu pour le ressort de compression (30).

10. Pièce de col d'essai selon l'une quelconque des revendications précédentes, **caractérisée en ce qu'**une fenêtre d'inspection (23) est prévue, laquelle est disposée de sorte que, lorsque le corps de tige (60) est inséré, elle indique un champ d'identification (65) associé à la position d'encliquetage respective, dans laquelle de préférence le champ d'identification (65) est pourvu d'un marquage pour une mesure de taille et/ou de profondeur.

11. Pièce de col d'essai selon l'une quelconque des revendications précédentes, **caractérisée en ce que** le corps de tige (60) séparé est conçu sous la forme d'une partie d'un outil de brochage, en particulier d'un alésoir (6), dans laquelle de préférence, un raccord standard pour des instruments chirurgicaux est réalisé à l'extrémité supérieure de l'outil de brochage, en particulier l'alésoir (6), de préférence sous la forme d'un raccord Hudson, et/ou l'outil de brochage est conçu en deux parties avec une partie proximale et une partie distale (68, 69), qui sont reliées de manière imperdable selon une articulation angulaire et de manière solidaire en rotation.

12. Instruments comprenant une pièce de col d'essai selon l'une quelconque des revendications précédentes ainsi qu'un instrument d'insertion (7), qui est pourvu à son extrémité distale d'un accouplement rapide (4) libérable pour la réception angulairement solidaire de la pièce de col d'essai (1).

13. Instruments selon la revendication 12, **caractérisés en ce que** l'instrument d'insertion (7) présente à son extrémité avant un module de préhension (44) avec une fourche de réception fixe (45) et un corps de serrage (47) disposé de manière mobile longitudinalement sur le fond de la fourche de réception, qui est réalisé de préférence également en forme de fourche.

14. Instruments selon la revendication 13, **caractérisés en ce que** le corps de serrage est actionné au moyen d'un organe coulissant (48), qui fait de préférence partie d'un dispositif de fixation, et/ou l'organe coulissant (48) est conçu sous la forme d'une barre élastique.

15. Instruments selon la revendication 14, **caractérisés en ce que** le dispositif de fixation est conçu de manière bistable, de préférence au moyen d'un mécanisme à détente brusque (49), plus préférablement avec deux butées pour une position de préhension et de libération.

16. Groupe d'instruments pour implanter une prothèse dans un os de forme allongée (os long) comprenant :
un alésoir, qui est réalisé pour être inséré depuis une extrémité de l'os long dans une cavité de l'os le long d'un axe long de l'os long et pour enlever de la matière osseuse, et qui présente une tête d'entraînement sur une partie de tête, ainsi qu'une pièce de col d'essai selon l'une quelconque des revendications 1-11,
**caractérisé en ce que**
la tête d'entraînement est conçue sous la forme d'un polygone, la pièce de col d'essai est conçue sous la forme d'une pièce rapportée enfichable, qui peut être enfichée avec sa zone de fixation par coopération de formes sur la tête d'entraînement et un dispositif d'encliquetage est prévu, qui comprend plusieurs niveaux d'encliquetage pour une disposition de la pièce de col d'essai à différentes hauteurs sur l'alésoir, dans lequel la pièce de col d'essai est arrêtée dans le niveau d'encliquetage de manière à ne pas pouvoir basculer,
et un dispositif de blocage est prévu, lequel fixe la pièce de col d'essai dans le niveau d'encliquetage respectif.
